# EUROPÄISCHE PATENTSCHRIFT

(11) **EP 1 430 846 B1**
(45) Veröffentlichungstag und Bekanntmachung des Hinweises auf die Patenterteilung: **02.08.2006**
(21) Anmeldenummer: 03026054.1
(22) Anmeldetag: 12.11.2003
(51) Int. Cl.: A61B 17/86, A61B 17/70

(54) **Knochenschraube für die Wirbelsäulen- oder Knochenchirurgie**
Bone screw for spinal or orthopaedic surgery
Vis à os pour chirurgie rachidienne ou orthopédique

(30) Priorität: 20.12.2002 DE 10260222
(43) Veröffentlichungstag der Anmeldung: 23.06.2004
(73) Patentinhaber: BIEDERMANN MOTECH GmbH, 78054 VS-Schwenningen (DE)
(72) Erfinder: Biedermann, Lutz, 78048 VS-Villingen (DE); Harms, Jürgen, 76227 Karlsruhe (DE)
(74) Vertreter: Hofer, Dorothea

(56) Entgegenhaltungen:
- WO-A-02/38054
- WO-A-98/35636
- WO-A-98/48738
- DE-A- 10 115 014
- DE-A- 19 540 180
- DE-A- 19 949 285
- US-A- 5 906 616
- US-A- 6 048 343
- US-B1- 6 391 058
- PATENT ABSTRACTS OF JAPAN vol. 1995, no. 05, 30. Juni 1995 (1995-06-30) -& JP 07 051292 A (YOSHIHIRO KISHIGAMI), 28. Februar 1995 (1995-02-28)

## Beschreibung

Die Erfindung betrifft eine Knochenschraube für die Wirbelsäulen- oder Knochenchirurgie und ein Implantat mit einer solchen Knochenschraube.

Eine Knochenschraube nach dem Oberbegriff des Patentanspruchs 1 und ein Implantat mit einer solchen einer Knochenschraube ist aus der WO 02/38054 bekannt. Mit der bekannten Knochenschraube ist es möglich, bei einer Knochenfraktur Teile des Knochen miteinander fest zu verbinden, indem die Knochenschraube als Zugelement und gleichzeitig als Fusionselement wirkt, wobei die Ausnehmungen in der Wandung das Einwachsen von Knochenmaterial oder Gefäßen erlauben.

Bei der bekannten Knochenschraube sind jedoch die Wendelspitzen des Knochengewindes an den Stellen unterbrochen, an denen Ausnehmungen in der Wandung gebildet sind. Dadurch werden Zähne gebildet, die beim Einschrauben in den Knochen eine Fräswirkung haben und den Knochen beschädigen können. Insbesondere für z. B. durch Osteoporose geschwächte Knochen kann die Verwendung der bekannten Knochenschraube zu Problemen beim Einschrauben führen.

Aus der DE 199 49 285 C2 ist eine Knochenschraube bekannt, die einen Schaft mit einem Knochengewinde, eine Spitze und einen Kopf aufweist, die einstückig miteinander ausgebildet sind. Die Knochenschraube weist ferner eine sich durch den Kopf und den Schaft erstreckende koaxiale Sacklochbohrung auf, von der aus sich eine Mehrzahl von radialen Bohrungen durch die Schraubenwand hindurch erstrecken. Die radialen Bohrungen sind jeweils zwischen den Gewindeflanken des Knochengewindes angeordnet. Die Bohrungen dienen als Kanal für die Einspritzung und Verteilung von Knochenzement. Ein Einwachsen von Knochenmaterial oder Gefäßen in die Bohrungen ist daher nicht möglich.

Aus der DE 195 40 180 A1 ist eine Schraubverbindung für ein Knochengelenk bekannt, die eine Hülse beinhaltet, welche am Ende ein Innengewinde zum Einschrauben eines Verschlußteiles erlaubt. Die Hülse weist ein Knochengewinde auf mit Zwischenräumen, die ins Innere der Hülse einmünden.

Aus der WO 98/35636 ist ein hohles Implantat bekannt, welches zylindrisch ausgebildet ist, mit einem im wesentlichen geschlossenen Ende und einem Innengewinde am anderen Ende, in das eine Verschlußkappe eingeschraubt werden kann. Das Implantat hat ein Knochengewinde an der Außenseite und eine Mehrzahl von Ausnehmungen in dem Knochengewindeabschnitt.

Es ist Aufgabe der Erfindung, eine verbesserte Knochenschraube der eingangs beschriebenen Art und ein dieses verwendendes Implantat bereitzustellen, das vielseitig einsetzbar ist.

Die Aufgabe wird gelöst durch eine Knochenschraube nach Anspruch 1 bzw. durch ein Implantat nach Anspruch 8. Weiterbildungen der Erfindung sind in den Unteransprüchen angegeben.

Weitere Merkmale und Zweckmäßigkeiten der Erfindung ergeben sich aus der Beschreibung von Ausführungsbeispielen anhand der Figuren. Von den Figuren zeigen:
- Fig. 1:: eine Seitenansicht einer ersten Ausführungsform der Erfindung des rohrförmigen Elements der Knochenschraube ;
- Fig. 2:: eine vergrößerte teilgeschnittene Darstellung des rohrförmigen Elements von Fig. 1;
- Fig. 3:: eine vergrößerte teilgeschnittene Darstellung einer Abwandlung des rohrförmigen Elements von Fig. 1; und

Aus der US 6,391,058 B1 ist ein Zwischenwirkel implantat bekannt, welches einen hohlen zylindrisch Körper mit einem Auβengeweinde beinhaltet, wobei Öffnungen in der der Wandung vorgesehen sind. In einer Ausführungsform sind die Öffnungen so angeordnet, daβ das Gewinde nicht durchbrochen wird.
- Fig. 4:: Ausführungsbeispiele von Implantaten unter Verwendung des rohrförmigen Elements von Fig. 1;
- Fig. 5:: weitere Ausführungsbeispiele von Implantaten unter Verwendung des rohrförmigen Elements von Fig. 1;
- Fig. 6a) bis c):: weitere Ausführungsbeispiele von Implantaten unter Verwendung des rohrförmigen Elements von Fig. 1
- Fig. 7:: eine zweite Ausführungsform des rohrförmigen Elements.

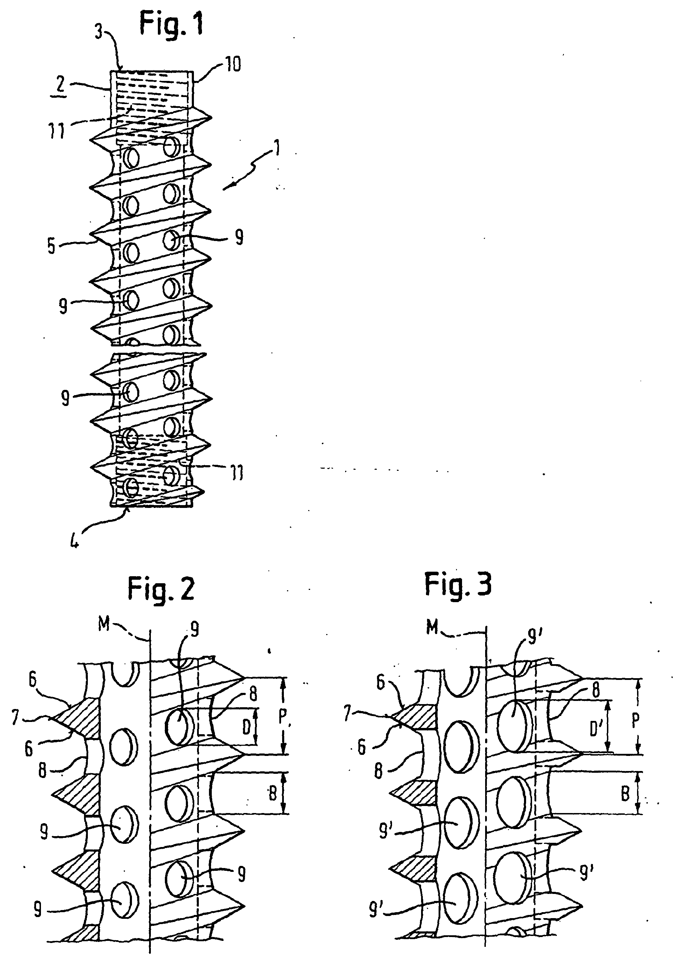

Wie am besten aus Fig. 1 und 2 ersichtlich ist, ist eine erste Ausführungsform des Elements 1 der erfindungsgemäßen Knochenschraube aus einem zylindrischen Rohr 2 mit einem ersten Ende 3 und einem diesem gegenüberliegenden zweiten Ende 4 ausgebildet. Das Rohr 2 weist an seiner Außenwand einen Knochengewindeabschnitt 5 mit einem Knochengewinde zum Einschrauben in einen Knochen auf. Das Knochengewinde ist als selbstschneidendes Gewinde ausgebildet und hat in bekannter Weise Gewindeflanken 6, eine Wendelspitze 7, einen Gewindegrund 8 mit einer Breite B und eine Gewindesteigung P. In wenigstens dem Knochengewindeabschnitt 5 weist die Wandung des Elements 1 eine Mehrzahl von Ausnehmungen 9 mit kreisförmigem Querschnitt auf. Die Ausnehmungen 9 sind so angeordnet, daß ihre Mitte jeweils im Gewindegrund 8 liegt und der Durchmesser einer jeden Ausnehmung 9 ist kleiner, als die Gewindesteigung P und insbesondere nicht größer als die Breite B des Gewindegrunds, so daß in dem in Fig. 1 und 2 dargestellten Ausführungsbeispiel die Ausnehmungen 9 vollständig im Gewindegrund 8 liegen und sich nicht in die Flanken 6 erstrecken. Es sind in jedem Gewindegang im Gewindegrund 8 eine Mehrzahl von auf der Schraubenlinie gleichmäßig beabstandeten Ausnehmungen 9 vorgesehen, so daß sich in axialer Richtung gesehen jeweils die Ausnehmungen eines Gewindegangs über den Ausnehmungen des darunterliegenden Gewindegangs befinden.

Wie insbesondere aus Fig. 1 ersichtlich ist, weist das Element 1 angrenzend an das erste Ende einen knochengewindefreien Abschnitt 10 mit einer glatten Außenwand auf, in der keine Ausnehmungen gebildet sind. Ferner ist bei dem gezeigten Ausführungsbeispiel angrenzend an das erste Ende 3 und angrenzend an das zweite Ende 4 des Rohrs ein Innengewindeabschnitt 11 gebildet, der zum Verbinden mit später beschriebenen Elementen eines Implantats dient.

Die Länge des Rohres 2 entspricht der Schaftlänge einer für die jeweilige Anwendung zu verwendenden Knochenschraube. Das Element besteht aus einem körperverträglichen Material, z. B. Titan oder Edelstahl.

Die in Fig. 3 gezeigte abgewandelte Ausführungsform unterscheidet sich von der in den Figuren 1 und 2 dargestellten Ausführungsform dadurch, daß der Durchmesser D' der Ausnehmung 9' größer ist, als die Breite des Gewindegrunds 8, so daß sich die Ausnehmungen 9' in die Flanken 6 des Knochengewindes hineinerstrecken, ohne jedoch die Wendelspitze 7 zu durchbrechen. Dadurch ist es möglich, die Ausnehmungen größer zu bilden, um eine bessere Fusionswirkung mit dem Knochen zu erzielen, eine Entstehung von Zähnen mit Fräswirkung beim Einschrauben wird jedoch verhindert, da die Schneidspitze des Gewindes unversehrt bleibt.

In einer nicht dargestellten Abwandlung der in den Figuren 1 und 2 gezeigten Ausführungsformen sind alle oder ein Teil der Ausnehmungen 9, 9' an der Außenseite der Wandung mit einer Ansenkung versehen, die eine Oberflächenrauhigkeit bildet, welche ein Ein- bzw. Anwachsen erleichtert. Der Durchmesser dieser Ansenkung in Schraubenachsrichtung ist jedoch kleiner als die Gewindesteigung P, so daß die Wendelspitze 7 unversehrt ist.

In einer weiteren Abwandlung sind die Ausnehmungen oval oder rautenförmig. Entscheidend ist, daß sie im Gewindegrund derart angeordnet sind und so bemessen sind, daß die Schneidspitze des Knochengewindes nicht verletzt wird. In einer weiteren Abwandlung sind nicht in jedem Gewindegang Ausnehmungen vorgesehen.

In einer weiteren Abwandlung erstreckt sich der Knochengewindeabschnitt 5 über die gesamte Länge des Rohrs 2. Das Innengewinde 11 kann sich ebenfalls über die gesamte Länge erstrecken. Alternativ kann das Innengewinde 11 auch nur an einem Ende in einem Abschnitt oder gar nicht vorgesehen sein. Die Verbindung mit den weiteren Implantatteilen erfolgt im Fall, daß kein Innengewinde vorgesehen ist, z.B. über Paßsitz.

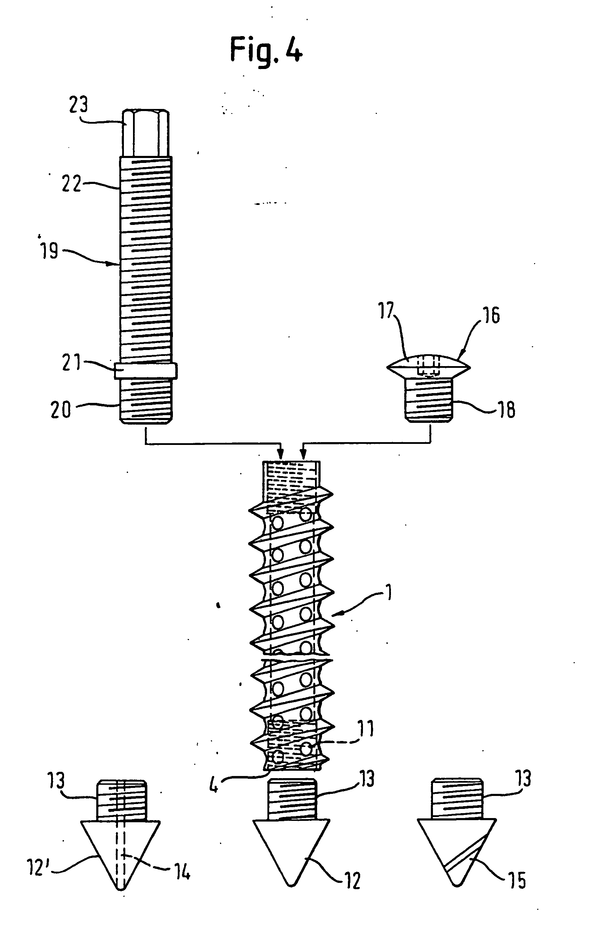

In den in den Figuren 4 bis 6 gezeigten erfindungsgemäßen Ausführungsbeispielen für Implantate ist das rohrförmige Element 1 jeweils ein Bestandteil des Implantats. Das rohrförmige Element ist an seinem zweiten Ende 4 in einer Ausführungsform mit einer Spitze 12 verbindbar, die den eigentlichen Spitzenteil und einen Schaft 13 umfaßt, der ein mit dem Innengewinde 11 des rohrförmigen Elements zusammenwirkendes Außengewinde aufweist. In einer Abwandlung weist die Spitze 12' eine koaxiale durchgehende Bohrung 15 auf, die einen Kanal zum Einbringen von Wirkstoffen an die Position, an der das Implantat verankert werden soll, bildet. In einer weiteren Abwandlung ist die Spitze als selbstschneidende Spitze 15 mit oder ohne durchgehende koaxiale Bohrung ausgebildet.

In einem ersten Ausführungsbeispiel ist das Implantat als Knochenschraube mit einem Kopf 16 ausgebildet, der den eigentlichen Kopfteil 17 mit einem Schlitz oder einem Innensechskant und einen Gewindeschaft 18 mit einem Außengewinde, das mit dem Innengewinde 11 des rohrförmigen Elements 1 zusammenwirkt, aufweist.

Im Betrieb wird ein rohrförmiges Element passender Länge ausgewählt oder ein entsprechend ausgebildetes Rohr auf die passende Länge gekürzt. Dann wird die Spitze mit dem rohrförmigen Element 1 fest verschraubt. Anschließend wird entweder gleich der Kopf aufgeschraubt und die Knochenschraube in den Knochen eingeschraubt. In diesem Fall dient der durch das rohrförmige Element gebildete Hohlraum zum Einwachsen von Knochenmaterial oder Gefäßen durch die Ausnehmungen 9 bzw. 9'. Alternativ wird entweder Knochenmaterial oder ein Wirkstoff in das rohrförmige Element 1 eingebracht und der Kopf aufgeschraubt. Dann wird die Knochenschraube zusammen mit dem eingebrachten Material in den Knochen eingeschraubt.

Aufgrund der Tatsache, daß die Wendelspitze des Knochengewindes nicht durch die Ausnehmungen unterbrochen ist, sondern unversehrt ist, erfolgt ein weiches Einschrauben ohne Fräseffekt, wodurch eine über das Einschrauben hinausgehende Beschädigung des Knochens nicht auftritt.

Als Instrument zum Einschrauben des rohrförmigen Elements 1 in den Knochen ist ein in Fig. 4 gezeigtes Gewindeelement 19 vorgesehen, das einen ersten Abschnitt 20 mit einem mit dem Innengewinde 11 zusammenwirkendes Außengewinde zum Einschrauben in das rohrförmige Element 1, einen Anschlag in Form einer Schulter 21 und einen daran angrenzenden Griffabschnitt 22 mit Außensechskant 23 aufweist. Der Griffabschnitt 22 kann mit oder ohne Außengewinde ausgebildet sein und seine Länge ist so bemessen, daß das rohrförmige Element 1 mit aufgeschraubter Spitze an einer gewünschten Stelle im Knochen positionierbar ist. In einer nicht dargestellten Abwandlung weist das Gewindeelement 19 einen durchgehenden koaxialen Kanal auf.

Im Betrieb wird das Gewindeelement 19 auf das rohrförmige Element 1 aufgeschraubt und dieses mittels des Gewindelements samt Spitze im Knochen versenkt. Anschließend wird das Gewindeelement wieder herausgeschraubt.

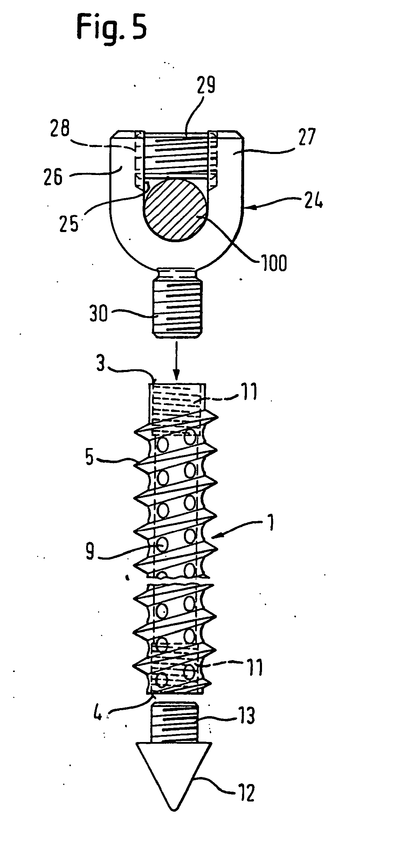

Bei dem in Fig. 5 gezeigten Ausführungsbeispiel besteht das Implantat aus einem Schraubenelement, das durch das rohrförmige Element 1 mit aufgeschraubter Spitze 12, 12' oder 15, wie zuvor beschrieben, gebildet ist, und aus einem mit dem Schraubenelement monoaxial verbindbaren Aufnahmeteil 24 zum Aufnehmen eines Stabs 100, der zum Verbinden mehrerer derartiger Implantate vorgesehen ist. Das Aufnahmeteil 24 ist im wesentlichen zylindrisch ausgebildet und weist eine Ausnehmung 25 mit U-förmigem Querschnitt auf, die gerade so groß bemessen ist, daß der Stab 100 einlegbar ist und in den Grund der Ausnehmung passt. Durch die U-förmige Ausnehmung 25 sind zwei freie Schenkel 26, 27 gebildet. Angrenzend an das freie Ende weisen die Schenkel 26, 27 ein Innengewinde 28 auf, welches mit einem entsprechenden Außengewinde einer zwischen die Schenkel einzuschraubenden Innenschraube 29 zum Fixieren des Stabs 100 zusammenwirkt. An seinem dem freien Ende abgewandten Ende weist das Aufnahmeteil 24 einen Gewindeschaft 30 zum Einschrauben in das rohrförmige Element 1 auf.

Im Betrieb wird vorzugsweise das Implantat zuerst vollständig zusammengesetzt, wobei das rohrförmige Element wenn es erforderlich ist, mit Wirkstoffen oder Knochenmaterial gefüllt ist. Anschließend wird das Implantat in den Knochen eingeschraubt. Dann erfolgt die Verbindung über den Stab zu einem oder mehreren anderen derartigen Implantaten. In korrekter Position wird anschließend der Stab über die Innenschraube fixiert. Das Implantat ist besonders geeignet für die Anwendung an der Wirbelsäule. Durch die durchgehende Wendelspitze des Knochengewindes des rohrförmigen Elements werden die Wirbel beim Einschrauben nicht zusätzlich beschädigt.

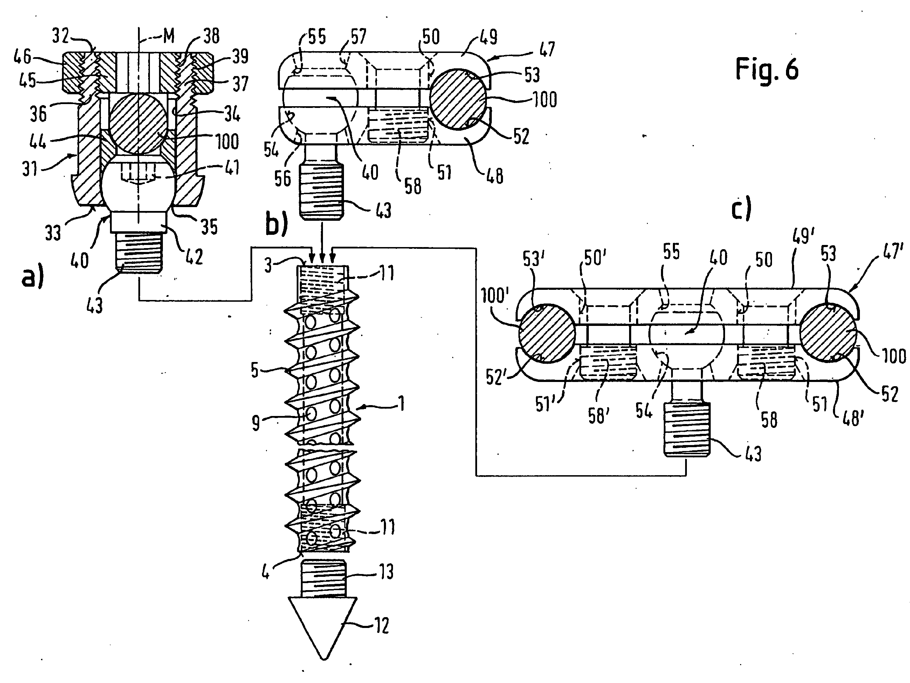

Die in Fig. 6 gezeigten weiteren Ausführungsbeispiele von Implantaten, sind ebenfalls Implantate bei denen ein aus dem rohrförmigen Element 1 und der Spitze 12, 12' oder 15 gebildetes Schraubenelement mit einem Aufnahmeteil zur Aufnahme eines Stabs 100 verbunden ist. Im Unterschied zu dem Ausführungsbeispiel nach Fig. 5 ist die Verbindung mit dem Stab jedoch polyaxial.

In dem in Fig. 6a) gezeigten Ausführungsbeispiel ist das Aufnahmeteil 31 zylindrisch ausgebildet mit einem ersten Ende 32 und einem gegenüberliegenden zweiten Ende 33. Koaxial zur Mittenschse M ist eine sich von dem ersten Ende 32 aus erstreckende erste koaxiale Bohrung 34 vorgesehen, die sich bis zu einem vorbestimmten Abstand von dem zweiten Ende 33 hin erstreckt. An dem zweiten Ende 33 ist eine zweite Bohrung 35 vorgesehen, die deren Durchmesser kleiner als der Durchmesser der ersten Bohrung ist und die sich zur ersten Bohrung hin in einem hohlkugelsegmentförmigen Abschnitt erweitert. Das Aufnahmeteil 31 weist ausgehend von dem ersten Ende 32 eine sich senkrecht zu Längsachse erstreckende U-förmige Ausnehmung durch die zwei freie Schenkeln 36, 37 gebildet sind. Angrenzend an das erste Ende 32 weisen die Schenkel einen Innengewinde 38 auf. Ferner ist an der Außenseite der Schenkel ein Außengewinde 39 vorgesehen.

Zum Verbinden des Schraubenelements mit dem Aufnahmeteil 31 ist ein kugelsegmentförmiger Schraubenkopf 40 vorgesehen, dessen Kugelradius im wesentlichen gleich dem Radius des hohlkugelsegmentförmigen Abschnitts des Aufnahmeteils 31 ist und der in lose eingesetztem Zustand in dem Aufnahmeteil schwenkbar ist. Der Kopf 40 weist ferner an seinem zu dem ersten Ende 32 des Aufnahmeteils 31 hin zu richtenden abgeflachten Ende eine Ausnehmung 41 zum Ineingriffbringen mit einem Schraubendreher auf. An seinem gegenüberliegenden Ende besitzt der Schraubenkopf 40 einen zylindrischen Hals 42 mit einem Außendurchmesser, der dem Außendurchmesser des rohrförmigen Elements 1 entspricht. Von dem Hals erstreckt sich ein Ansatz 43 mit einem Außengewinde mit dem der Schraubenkopf 40 in das rohrförmige Element einschraubbar ist.

Zum Fixieren des Kopfes 40 in dem Aufnahmeteil und damit der Winkelstellung des Schraubenelements ist ein Druckelement 44 vorgesehen, welches zylindrisch ausgebildet ist, so daß es in dem Aufnahmeteil verschiebbar ist und welches an seinem einen Ende eine sphärische Ausnehmung zur Aufnahme eines Abschnitts des Schraubenkopfs und an seinem gegenüberliegenden Ende eine U-förmige Ausnehmung zur Aufnahme des Stabs aufweist. Das Druckelement 44 weist ferner eine koaxiale Bohrung auf, damit das Schraubenelement bei eingesetztem Druckelement einschraubbar ist. Zum Fixieren von Kopf 40 und Stab 100 ist eine zwischen die Schenkel 36, 37 einschraubbare Innenschraube 45 vorgesehen. Zur Sicherung der Fixierung ist eine auf das Aufnahmeteil 31 aufschraubbare Sicherungsmutter 46 vorgesehen.

Im Betrieb wird zuerst das aus Spitze 12, gegebenenfalls mit Knochenmaterial oder einem Wirkstoff gefüllte rohrförmigem Element 1 und Schraubenkopf 40 bestehende Schraubenelement zusammengesetzt. Anschließend werden das Schraubenelement und das Druckelement in das Aufnahmeteil eingeführt und das Schraubenelement in den Knochen oder den Wirbel eingeschraubt. Die Verbindung mit anderen Implantaten über den Stab erfolgt in bekannter Weise.

Die Kopf- und Stabfixierung ist nicht auf das beschriebene beschränkt, sondern es kann jede Kopf- und Stabfixierung bekannter Polyaxialschrauben verwendet werden.

In dem in den Figuren 6b) und 6c) gezeigten Ausführungsbeispiel erfolgt die polyaxiale Verbindung mit dem Stab 100 nicht in Richtung der Schraubenachse, wie bei dem Beispiel gemäß Fig. 6a), sondern seitlich zur Schraubenachse versetzt.

Das Implantat gemäß Fig. 6b) umfaßt ein aus dem rohrförmigen Element 1, der Spitze 12 und dem in Fig. 6a) gezeigten kugelsegmentförmigen Kopf 40 bestehendes Schraubenelement sowie eine dem Kopf 40 aufnehmende zweiteilige Fassung 47 mit einem dem rohrförmigen Element 1 zugewandten Unterteil 48 und einem dem rohrförmigen Element 1 abgewandten Oberteil 49, die zusammen den Stab 100 umfassen. Das Oberteil 49 und das Unterteil 48 sind identisch ausgebildet und spiegelbildlich zueinander angeordnet. Sie weisen eine zentrale Bohrung 50, 51 auf, die mit einem Innengewinde versehen ist und auf der dem jeweils anderen Teil 48, 49 abgewandten Oberfläche eine Senkbohrung aufweist. Seitlich der Bohrung 50, 51 ist in einem Abstand von dieser eine zu dem jeweils anderen Teil 48, 49 hin zylindersegmentförmig ausgebildete Ausnehmung 52, 53 zum Halten des Stabs vorgesehen. Auf der anderen Seite der Bohrung 50 weisen das Unterteil 48 und das Oberteil 49 auf der dem jeweils anderen Teil zugewandten Seite eine kugelsegmentförmige Ausnehmung 54, 55 zum Halten des Schraubenkopfs 40 auf. Auf der dem anderen Teil 48, 49 abgewandten Oberfläche schließt sich koaxial zu der Ausnehmung 54, 55 eine nach außen zunehmende Ausnehmung 56, 57 an.

Unterteil 48 und Oberteil 49 der Fassung sind durch eine Schraube 58 miteinander verbunden, die in das Innengewinde des Oberteils einführbar ist, und in das Innengewinde des Unterteils einschraubbar ist. In ihrem durch das Oberteil 49 geführten Teil hat die Schraube 58 einen Durchmesser, der kleiner ist, als der Durchmesser des Innengewindes des Oberteils und weist in ihrem durch das Unterteil geführten Teil ein mit dem Innengewinde des Unterteils zusammenwirkendes Außengewinde auf. Die zylindersegmentförmigen Ausnehmungen 52, 53 und die kugelsegmentförmigen Ausnehmungen 54, 55 sind so dimensioniert und so zueinander angeordnet, daß in dem Zustand in dem der Stab und der Kopf gehalten sind, das Unterteil 48 und das Oberteil 49 parallel zueinander ausgerichtet sind und einen Abstand voneinander besitzen.

Im Betrieb wird erst das Schraubenelement zusammengesetzt. Das Oberteil und das Unterteil der Fassung sind durch Losedrehen der Schraube 58 um 90 Grad gegeneinander verdreht, so daß das Schraubenelement in das Unterteil 48 einführbar ist. Das Schraubenelement wird eingeführt, bis sein Kopf 40 in der kugelsegmentförmigen Ausnehmung 54 anliegt. Anschließend wird es in den Knochen eingeschraubt. Dann wird der Stab 100 aufgenommen und das Oberteil 49 um 90 Grad zum Fassen des Stabs gedreht. Nach erfolgter Justierung der Winkelstellung des Schraubenkopfs 40 in der Fassung und der nPosition des Stabs erfolgt ein Fixieren durch Festziehen der Schraube 58.

Das Implantat ist insbesondere für die Fixierung von Frakturen am Becken und an Röhrenknochen geeignet.

Das in Fig. 6c) dargestellte Ausführungsbeispiel unterscheidet sich von dem in Fig. 6b) dargestellten Ausführungsbeispiel dadurch, daß die Fassung 47' zum Erfassen von zwei Stäben 100, 100' ein in sich symmetrisch ausgebildetes Unterteil 48' und Oberteil 49' aufweist. Das Unterteil 48' und das Oberteil 49' dabei symmetrisch zu einer durch die Mittellinie der Stäbe 100, 100' und den Mittelpunkt des kugelsegmentförmigen Kopfs 40 der Schraube definierten Ebene ausgebildet und weisen jeweils zwei Bohrungen 50, 50', 51, 51', zwei zylindersegmentförmige Ausnehmungen 52, 52' bzw. 53, 53' auf. Zum Fixieren sind zwei Fixierschrauben 58, 58' vorgesehen. Der Betrieb ist analog zu dem vorherbeschriebenen Ausführungsbeispiel, nur daß zwei Stäbe zu fixieren sind.

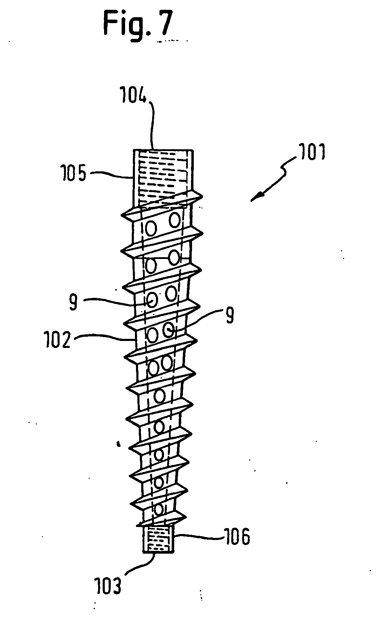

In einer in Fig. 7 gezeigten zweiten Ausführungsform ist das rohrförmige Element 101 nicht insgesamt zylindrisch ausgebildet, sondern weist einen mittleren konischen Knochengewindeabschnitt 102 auf, der sich in Richtung zu dem mit der Spitze zu verbindenden Ende 103 des Elements verjüngt. Angrenzend an den konischen Abschnitt erstreckt sich beidseitig des konischen Abschnitts bis zu den einander gegenüberliegenden Enden 103, 104 jeweils ein zylindrischer Abschnitt 105, 106 mit einem Innengewinde zum Verbinden mit der Spitze an einem Ende bzw. mit einem Kopf, einem Gewindeelement, oder einem Aufnahmeteil wie zuvor beschrieben am anderen Ende.

In einer Abwandlung ist der mit der Spitze zu verbindende zylindrische Abschnitt 106 nicht vorgesehen, sondern das freie Ende des konischen Knochengewindeabschnitts wirkt selbst als Spitze.

## Patentansprüche

1. Knochenschraube mit einem rohrförmigen Element für die Wirbelsäulen- oderKnochenchirurgie mit einem Knochengewindeabschnitt (5; 102) mit einem Knochengewinde und mit einer Mehrzahl von Ausnehmungen (9, 9') in dem Knochengewindeabschnitt (5; 102), wobei wenigstens ein Ende des Elements (1; 101) zur Aufnahme eines Kopfes bzw. einer Spitze ausgebildet ist, wobei eine an einem Ende mit dem rohrförmigen Element (1; 101) lösbar verbundene Spitze (12, 12', 15) vorgesehen ist, **dadurch gekennzeichnet daβ**, die Ausnehmungen im Gewindegrund (8) des Knochengewindes angeordnet sind und so bemessen sind, daß die Wendelspitze (7) des Knochengewindes unversehrt ist.

2. Knochenschraube nach Anspruch 1, **dadurch gekennzeichnet, daß** wenigstens eine Ausnehmung so bemessen ist, daß ein größter Durchmesser der Ausnehmung (9) kleiner ist als der Abstand der Gewindeflanken (6) am Gewindegrund (8).

3. Knochenschraube nach Anspruch 1 oder zwei, **dadurch gekennzeichnet, daß** wenigstens eine Ausnehmung (9') so bemessen ist, daß ein größter Durchmesser der Ausnehmung (9) größer ist als der Abstand der Gewindeflanken (6) am Gewindegrund (8), wobei sich die Ausnehmung dann in wenigstens eine der Flanken (6) erstreckt.

4. Knochenschraube nach einem der Ansprüche 1 bis 3, **dadurch gekennzeichnet, daß** wenigstens eine der Ausnehmungen (9, 9') an der Außenseite der Wandung eine Ansenkung aufweist.

5. Knochenschraube nach einem der Ansprüche bis 4, **dadurch gekennzeichnet, daß** in jedem Gewindegang eine Mehrzahl von gleichmäßig beabstandeten Ausnehmungen vorgesehen ist.

6. Knochenschraube nach einem der Ansprüche 1 bis 5, **dadurch gekennzeichnet, daß** an wenigstens einem Ende ein Innengewindeabschnitt (10,11; 105, 106) zum Verschrauben mit einem Kopf (16; 24; 40) oder einer Spitze (12, 13) vorgesehen ist.

7. Knochenchraube nach einem der Ansprüche 1 bis 6, **dadurch gekennzeichnet, daß** der Knochengewindeabschnitt (102) konisch ausgebildet ist.

8. Knochenschraube nach einem des Ansprüche 1 bis 7, **dadurch gekennzeichnet, daß** ein mit dem anderen Ende des rohrförmigen Elements (1; 101) lösbar verbundener Kopf (16; 19; 24; 40, 41, 47, 47') vorgesehen ist.

9. Knochenschraube nach Anspruch 8, **dadurch gekennzeichnet, daß** der Kopf als Schraubenkopf (17; 40) ausgebildet ist.

10. Knochenschraube nach Anspruch 8, **dadurch gekennzeichnet, daß** der Kopf als im wesentlichen zylindrisches Gewindeelement (19) ausgebildet ist.

11. Knochenschraube nach Anspruch 8, **dadurch gekennzeichnet, daß** der Kopf (24; 41; 47; 47') als Aufnahmeteil für einen mehrere Knochenschrauben verbindenden Stab (100, 100') ausgebildet ist.

12. Knochenschraube nach Anspruch 11, **dadurch gekennzeichnet, daß** der Kopf (24) und das rohrförmige Element (1; 101) monoaxial miteinander verbunden sind.

13. Knochenschraube nach Anspruch 11, **dadurch gekennzeichnet, daß** der Kopf (40,41; 40, 47; 40, 47') und das rohrförmige Element (1; 101) polyaxial miteinander verbunden sind.

## Claims

1. Bone screw with a tubular element for spine or bone surgery having a bone thread section (5; 102) with a bone thread and with a plurality of apertures (9, 9') in the bone thread section (5; 102), wherein at least one end of the element (1; 101) is formed for receiving a head or a tip,
wherein a tip (12, 12', 15) being detachably connected at one end to the tubular element (1; 101) is provided,
**characterized in that** the apertures are arranged in the root (8) of the bone thread and are dimensioned such that the crest (7) of the bone thread is intact.

2. Bone screw according to claim 1, **characterized in that** at least one aperture is dimensioned such that a largest diameter of the aperture (9) is smaller than the distance of the thread flanks (6) at the root of the thread (8).

3. Bone screw according to claim 1 or 2, **characterized in that** at least one aperture (9') is dimensioned such that a largest diameter of the aperture (9) is larger than the distance of the thread flanks (6) at the root of the thread (8), wherein the aperture then extends into at least one of the flanks (6).

4. Bone screw according to one of claims 1 to 3, **characterized in that** at least one of the apertures (9, 9') comprises a countersinking at the outside of the wall.

5. Bone screw according to one of claims 1 to 4, **characterized in that** in each turn of the thread a plurality of equally distanced apertures are provided.

6. Bone screw according to one of claims 1 to 5, **characterized in that** at least on one end an inner thread section (10, 11; 105, 106) for screwing-in a head (16; 24; 40) or a tip (12, 13) is provided.

7. Bone screw according to one of claims 1 to 6, **characterized in that** the bone thread section (102) is conically shaped.

8. Bone screw according to one of claims 1 to 7, **characterized in that** a head (16; 19; 24; 40, 41, 47, 47') being detachably connected to the other end of the tubular element (1; 101) is provided.

9. Bone screw according to claim 8, **characterized in that** the head is formed as screw head (17; 40).

10. Bone screw according to claim 8, **characterized in that** the head is formed as substantially cylindrical threaded element (19) .

11. Bone screw according to claim 8, **characterized in that** the head (24; 41; 47; 47') is formed as receiver member for a rod (100, 100') connecting several bone screws.

12. Bone screw according to claim 11, **characterized in that** the head (24) and the tubular element (1; 101) are monoaxially connected to each other.

13. Bone screw according to claim 11, **characterized in that** the head (40, 41; 40, 47; 40, 47') and the tubular element (1; 101) are polyaxially connected to each other.

## Revendications

1. Vis à os, comprenant un élément tubulaire pour la chirurgie rachidienne ou orthopédique, avec un tronçon de filetage à os (5 ; 102) avec un filetage à os et une pluralité d'évidements (9, 9') ménagés dans le tronçon de filetage à os (5 ; 102), sachant qu'au moins une extrémité de l'élément (1 ; 101) est réalisée pour recevoir une tête ou une pointe, où une pointe (12, 12', 15), reliée de façon désolidarisable à une extrémité à l'élément tubulaire (100 ; 101), est prévue, **caractérisée en ce que** les évidements sont disposés dans le fond de filetage (8) du filetage à os et dimensionnés de manière que la pointe d'enroulement (7) du filetage à os soit laissée intacte.

2. Vis à os selon la revendication 1, **caractérisée en ce qu'**au moins un évidement est dimensionné de manière qu'un diamètre maximal de l'évidement (9) soit inférieur à l'espacement des flancs de filetage (6) au fond de filetage (8).

3. Vis à os selon la revendication 1 ou 2, **caractérisée en ce qu'**au moins un évidement (9') est dimensionné de manière qu'un diamètre maximal de l'évidement (9) soit supérieur à l'espacement des flancs de filetage (6) au fond de filetage (8), l'évidement s'étendant alors en au moins l'un des flancs (6).

4. Vis à os selon l'une des revendications 1 à 3, **caractérisée en ce qu'**au moins l'un des évidements (9, 9') présente une fraisure sur la face extérieure de la paroi.

5. Vis à os selon l'une des revendications 1 à 4, **caractérisée en ce que**, dans chaque pas de filetage, est prévue une pluralité d'évidements espacés régulièrement.

6. Vis à os selon l'une des revendications 1 à 5, **caractérisée en ce qu'**en au moins une extrémité est prévu un tronçon de filetage intérieur (10, 11 ; 105, 106), pour vissage à une tête (16 ; 24 ; 40) ou à une pointe (12, 13).

7. Vis à os selon l'une des revendications 1 à 6, **caractérisée en ce que** le tronçon de filetage à os (102) est de forme conique.

8. Vis à os selon l'une des revendications 1 à 7, **caractérisée en ce qu'**une tête (16 ; 19 ; 24 ; 40, 41, 47, 47'), reliée de façon désolidarisable à l'autre extrémité de l'élément tubulaire (1 ; 101), est prévue.

9. Vis à os selon la revendication 8, **caractérisée en ce que** la tête est réalisée sous forme de tête de vis (17 ; 40).

10. Vis à os selon la revendication 8, **caractérisée en ce que** la tête est réalisée sous forme d'élément fileté (19) sensiblement cylindriques.

11. Vis à os selon la revendication 8, **caractérisée en ce que** la tête (24 ; 41 ; 47 ; 47') est réalisée sous forme de partie réceptrice, pour une barre (100, 100') reliant plusieurs vis à os.

12. Vis à os selon la revendication 11, **caractérisée en ce que** la tête (24) et l'élément tubulaire (1 ; 101) sont reliés ensemble de façon monoaxiale.

13. Vis à os selon la revendication 11, **caractérisée en ce que** la tête (40, 41 ; 40, 47 ; 40, 47') et l'élément tubulaire (1 ; 101) sont reliés ensemble de façon polyaxiale.
